# EUROPEAN PATENT APPLICATION

(11) **EP 4 201 920 A1**
(43) Date of publication of application: **28.06.2023**
(21) Application number: 21858382.1
(22) Date of filing: 19.08.2021
(51) Int. Cl.: C07C 67/58, C07C 69/653

(54) **METHOD FOR PURIFYING HALOGEN-CONTAINING (METH)ACRYLATE**

(30) Priority: 19.08.2020 JP 2020138907
(71) Applicant: DAIKIN INDUSTRIES, LTD., Osaka-shi, Osaka 530-0001 (JP)
(72) Inventor: MATSUURA, Makoto, Osaka-shi, Osaka 530-0001 (JP); TAKUBO, Seiji, Osaka-shi, Osaka 530-0001 (JP); HOSOKAWA, Moe, Osaka-shi, Osaka 530-0001 (JP); KISHIKAWA, Yosuke, Osaka-shi, Osaka 530-0001 (JP); GOTOU, Akihiro, Osaka-shi, Osaka 530-0001 (JP)
(74) Representative: Hoffmann Eitle
(86) International application number: PCT/JP2021/030449
(87) International publication number: WO 2022/039238

(57) **Abstract**

The present disclosure addresses the problem of providing a method for purifying a halogen-containing (meth)acrylic acid ester, the method being capable of removing an alcohol to a high degree. This problem can be solved by a method for purifying a compound represented by formula (1): 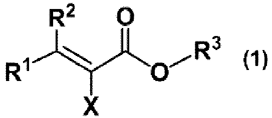 (wherein R¹ and R² are be the same or different and represent an alkyl group, a fluoroalkyl group, an aryl group optionally having one or more substituents, a halogen atom, or a hydrogen atom, R³ represents an alkyl group, a fluoroalkyl group, or an aryl group optionally having one or more substituents, and X represents a fluoroalkyl group or a halogen atom),
the method comprising step (A) of mixing a composition comprising the compound represented by formula (1) and a compound represented by formula (2): R⁴-OH (2)
(wherein R⁴ is an alkyl group, a fluoroalkyl group, or an aryl group optionally having one or more substituents) with (i) a salt and/or (ii) a specific organic solvent to obtain a mixture; and step (B) of separating the mixture into two or more phases that are different from each other in terms of the content of the compound represented by formula (1).

## Description

### Technical Field

The present disclosure relates to a method for purifying a halogen-containing (meth)acrylic acid ester.

### Background Art

Halogen-containing (meth)acrylic acid esters are useful as synthetic intermediates of pharmaceuticals (e.g., antibiotics), synthetic intermediates for sheath materials of optical fibers, synthetic intermediates of coating materials, synthetic intermediates of semiconductor resist materials, monomers of functional polymers, and the like.

When a halogen-containing (meth)acrylic acid ester is to be produced, a composition containing the desired halogen-containing (meth)acrylic acid ester may contain impurities. Examples of impurities include reaction solvents (e.g., alcohols), catalysts, bases, washing solvents (e.g., water), and the like.

Of these, water, for example, may hydrolyze halogen-containing (meth)acrylic acid esters and thus adversely affect their stability. A known method for removing such water is, for example, a method comprising bringing a composition containing a halogen-containing (meth)acrylic acid ester and water into contact with zeolite (Patent Literature (PTL) 1).

In addition, a known method for removing an alcohol is, for example, a method comprising bringing a composition containing a halogen-containing (meth)acrylic acid ester and an alcohol into contact with an acid anhydride (PTL 2).

### Citation List

### Patent Literature

PTL 1: JP2017-036272A
PTL 2: JP2017-036270A

### Summary of Invention

### Technical Problem

When a halogen-containing (meth)acrylic acid ester is to be produced, a composition containing the halogen-containing (meth)acrylic acid ester may contain alcohol as an impurity. Even if the composition is washed with water, the alcohol is not fully removed. When using a method comprising bringing the composition into contact with acid anhydride, there is room for improvement in view of a large amount of waste and increased cost due to the use of a large amount of acid anhydride.

An object of the present disclosure is to provide a method for purifying a halogen-containing (meth)acrylic acid ester, the method being capable of removing alcohol to a high degree.

### Solution to Problem

The present disclosure includes the following embodiments.

### Item 1.

A method for purifying a compound represented by formula (1): 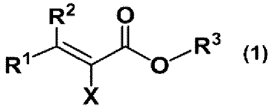 (wherein
R¹ and R² are the same or different and are an alkyl group, a fluoroalkyl group, an aryl group optionally having one or more substituents, a halogen atom, or a hydrogen atom,
R³ is an alkyl group, a fluoroalkyl group, or an aryl group optionally having one or more substituents, and
X is a fluoroalkyl group or a halogen atom), the method comprising
step (A) of mixing
   a composition comprising
   the compound represented by formula (1) and
   a compound represented by formula (2):

      R⁴-OH (2)

      wherein R⁴ is an alkyl group, a fluoroalkyl group, or an aryl group optionally having one or more substituents
      with
      (i) a salt,
      (ii) an organic solvent, with the proviso that the compound represented by formula (1) and the compound represented by formula (2) are excluded from the organic solvent, or
      (iii) the salt and the organic solvent
         to obtain a mixture; and
step (B) of separating the mixture into two or more phases that are different from each other in terms of the content of the compound represented by formula (1).

### Item 2.

The method according to Item 1, wherein the salt is at least one member selected from the group consisting of inorganic salts and organic salts.

### Item 3.

The method according to Item 1 or 2, wherein the salt is an inorganic salt.

### Item 4.

The method according to any one of Items 1 to 3, wherein the salt comprises at least one cation selected from the group consisting of metal cations, ammonium optionally having one or more substituents, pyridinium optionally having one or more substituents, imidazolium optionally having one or more substituents, and phosphonium optionally having one or more substituents.

### Item 5.

The method according to any one of Items 1 to 4, wherein the salt comprises at least one cation selected from the group consisting of monovalent metal cations and divalent metal cations.

### Item 6.

The method according to any one of Items 1 to 4, wherein the salt comprises a cation of NR⁴⁺ (wherein each R may be the same or different and is H or a C₁₋₁₀ organic group) .

### Item 7.

The method according to any one of Items 1 to 6, wherein the salt comprises at least one anion selected from the group consisting of sulfate ions, hydroxide ions, halide ions, and nitrate ions.

### Item 8.

The method according to any one of Items 1 to 5, wherein the salt is at least one member selected from the group consisting of LiCl, LiBr, LiI, NaI, and CaCl₂.

### Item 9.

The method according to any one of Items 1 to 8, wherein the salt is used in an amount of 0.1 to 10 moles per mole of the compound represented by formula (1).

### Item 10.

The method according to any one of Items 1 to 9, wherein the organic solvent is an aprotic solvent (with the proviso that the compound represented by formula (1) and the compound formula (2) are excluded from the aprotic solvent).

### Item 11.

The method according to any one of Items 1 to 10, wherein the organic solvent is an aprotic nonpolar solvent (with the proviso that the compound represented by formula (1) and the compound represented by formula (2) are excluded from the aprotic nonpolar solvent).

### Item 12.

The method according to any one of Items 1 to 10, wherein the organic solvent is at least one member selected from the group consisting of aliphatic hydrocarbons, aromatic hydrocarbons, halogenated hydrocarbons, ethers, esters (with the proviso that the compound represented by formula (1) is excluded from the esters), ketones, carbonates, and nitriles.

### Item 13.

The method according to any one of Items 1 to 10, wherein the organic solvent is at least one member selected from the group consisting of aromatic hydrocarbons and ethers.

### Item 14.

The method according to any one of Items 1 to 10, wherein the organic solvent is at least one member selected from the group consisting of C₅₋₁₆ alkane, C₅₋₁₀ cycloalkane, benzene optionally having at least one C₁₋₄ alkyl, C₁₋₆ haloalkane, benzene having at least one halogen atom, di (C₁₋₄ alkyl) ether, di (C₁₋₄ alkyl) ether of C₂₋₄ alkylene glycol, di (C₁₋₄ alkyl) ether of poly (C₂₋₄ alkylene glycol), 5-membered oxygen-containing heterocyclic rings, C₁₋₆ alkanoic acid C₁₋₄ alkyl esters, di (C₁₋₄ alkyl) ketone, C₂₋₄ alkylene carbonate, C₁₋₆ cyanoalkane, and benzene having at least one cyano group.

### Item 15.

The method according to any one of Items 1 to 10, wherein the organic solvent is at least one member selected from the group consisting of pentane, hexane, heptane, octane, nonane, decane, undecane, dodecane, tridecane, tetradecane, pentadecane, hexadecane, cyclopentane, cyclohexane, benzene, xylene, toluene, dichloromethane, dichloroethane, dichloropropane, chlorobutane, chloroform, chlorobenzene, dichlorobenzene, diethyl ether, diisopropyl ether, t-butyl methyl ether, dibutyl ether, monoglyme, diglyme, triglyme, 1,4-dioxane, tetrahydrofuran, ethyl acetate, butyl acetate, methyl ethyl ketone, acetone, ethylene carbonate, propylene carbonate, acetonitrile, and benzonitrile.

### Item 16.

The method according to any one of Items 1 to 15, wherein the organic solvent is used in an amount of 0.1 to 10 moles per mole of the compound represented by formula (1).

### Item 17.

The method according to any one of Items 1 to 16, wherein the step (A) is a step of mixing the composition with
(i) a salt, (ii) an organic solvent (with the proviso that the compound represented by formula (1) and the compound represented by formula (2) are excluded from the organic solvent), or (iii) the salt and the organic solvent, and
(iv) water
to obtain a mixture.

### Item 18.

The method according to Item 17, wherein the salt is used in an amount of 150 mg or more per mL of water.

### Item 19.

The method according to Item 17 or 18, wherein the salt is LiCl, LiBr, LiI, NaI, or CaCl₂;
when the salt is LiCl, the salt is used in an amount of 150 mg or more per mL of water;
when the salt is LiBr, the salt is used in an amount of 310 mg or more per mL of water;
when the salt is LiI, the salt is used in an amount of 480 mg or more per mL of water;
when the salt is NaI, the salt is used in an amount of 540 mg or more per mL of water; and
when the salt is CaCl₂, the salt is used in an amount of 450 mg or more per mL of water.

### Item 20.

The method according to any one of Items 1 to 19, further comprising step (C) of removing a phase that has the lowest content of the compound represented by formula (1) of the separated phases.

### Item 21.

The method according to any one of Items 1 to 20, which is performed at a temperature of -15 to 40°C.

### Item 22.

The method according to any one of Items 1 to 21, wherein R¹ is a hydrogen atom, an alkyl group, or a fluoroalkyl group.

### Item 23.

The method according to any one of Items 1 to 22, wherein R² is a hydrogen atom, an alkyl group, or a fluoroalkyl group.

### Item 24.

The method according to any one of Items 1 to 23, wherein R³ is an alkyl group.

### Item 25.

The method according to any one of Items 1 to 24, wherein R³ is a C₁₋₄ alkyl group.

### Item 26.

The method according to any one of Items 1 to 25, wherein R⁴ is an alkyl group.

### Item 27.

The method according to any one of Items 1 to 26, wherein R⁴ is a C₁₋₄ alkyl group.

### Item 28.

The method according to any one of Items 1 to 27, wherein X is a fluorine atom or a chlorine atom.

### Item 29.

A composition comprising
a compound represented by formula (1): 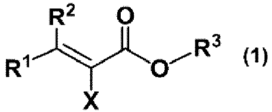 (wherein
R¹ and R² are the same or different and represent an alkyl group, a fluoroalkyl group, an aryl group optionally having one or more substituents, a halogen atom, or a hydrogen atom,
R³ represents an alkyl group, a fluoroalkyl group, or an aryl group optionally having one or more substituents, and
X represents a fluoroalkyl group or a halogen atom);
a compound represented by formula (2):

   R⁴-OH (2)

   (wherein R⁴ is an alkyl group, a fluoroalkyl group, or an aryl group optionally having one or more substituents); and
a salt, the content of the salt being 2 mass% or less.

### Item 29a.

The composition according to Item 29, wherein the salt is the salt recited in Item 2, 3, or 8.

### Item 29b.

The composition according to Item 29, wherein the salt comprises the cation recited in any one of Items 4 to 6 and/or the salt comprises the anion recited in Item 7.

### Item 29c.

The composition according to Item 29, 29a, or 29b, further comprising an organic solvent.

### Item 29d.

The composition according to Item 29c, wherein the organic solvent is the organic solvent of any one of Items 10 to 15.

### Item 29e.

The composition according to any one of Items 29 and 29a to 29d, wherein R¹ is a hydrogen atom, an alkyl group, or a fluoroalkyl group.

### Item 29f.

The composition of any one of Items 29 and 29a to 29e, wherein R² is a hydrogen atom, an alkyl group, or a fluoroalkyl group.

### Item 29g.

The composition according to any one of Items 29 and 29a to 29f, wherein R³ is an alkyl group.

### Item 29h.

The composition according to any one of Items 29 and 29a to 29g, wherein R³ is a C₁₋₄ alkyl group.

### Item 29i.

The composition according to any one of Items 29 and 29a to 29h, wherein R⁴ is an alkyl group.

### Item 29j.

The composition according to any one of Items 29 to 29a to 29i, wherein R⁴ is a C₁₋₄ alkyl group.

### Item 29k.

The composition according to any one of Items 29 to 29a to 29j, wherein X is a fluorine atom.

### Item 30.

A composition comprising
a compound represented by formula (1): 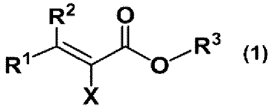 (wherein R¹ and R² are the same or different and represent an alkyl group, a fluoroalkyl group, an aryl group optionally having one or more substituents, a halogen atom, or a hydrogen atom),
R³ is an alkyl group, a fluoroalkyl group, or an aryl group optionally having one or more substituents, and
X is a fluoroalkyl group or a halogen atom); and
   at least one organic solvent selected from the group consisting of aliphatic hydrocarbons, aromatic hydrocarbons, halogenated hydrocarbons, ethers, esters (with the proviso that the compound represented by formula (1) is excluded from the esters), ketones, carbonates, and nitriles,
   the content of the organic solvent being 20 mass% or less.

### Item 30a.

The composition according to Item 30, further comprising a compound represented by formula (2):

R⁴-OH (2)

(wherein R⁴ is an alkyl group, a fluoroalkyl group, or an aryl group optionally having one or more substituents).

### Item 30b.

The composition according to Item 30 or 30a, wherein R¹ is a hydrogen atom, an alkyl group, or a fluoroalkyl group. Item 30c.

The composition according to Item 30, 30a, or 30b, wherein R² is a hydrogen atom, an alkyl group, or a fluoroalkyl group.

### Item 30d.

The composition according to any one of Items 30 and 30a to 30c, wherein R³ is an alkyl group.

### Item 30e.

The composition according to any one of Items 30 to 30a to 30d, wherein R³ is a C₁₋₄ alkyl group.

### Item 30f.

The composition according to any one of Items 30a to 30e, wherein R⁴ is an alkyl group.

### Item 30g.

The composition according to any one of Items 30a to 30f, wherein R⁴ is a C₁₋₄ alkyl group.

### Item 30h.

The composition according to any one of Items 30 to 30a to 30g, wherein X is a fluorine atom.

### Advantageous Effects of Invention

According to the present disclosure, there is provided a method for purifying a halogen-containing (meth)acrylic acid ester, the method being capable of removing alcohol to a high degree.

### Description of Embodiments

The above summary of the present disclosure is not intended to describe each disclosed embodiment or every implementation of the present disclosure.

The description of the present disclosure that follows more specifically exemplifies illustrative embodiments.

In several places throughout the present disclosure, guidance is provided through lists of examples, and these examples can be used in various combinations.

In each instance, the described list serves only as a representative group, and should not be interpreted as an exclusive list.

All of the publications, patents, and patent applications cited herein are incorporated herein by reference in their entirety.

### Terms

The symbols and abbreviations in this specification can be understood to have the meanings commonly used in the technical field of the present disclosure in the context of the present description, unless otherwise specified.

The term "comprising" used in this specification is intended to include "consisting essentially of" and "consisting of."

The steps, treatments, or operations described in this specification can be performed at room temperature, unless otherwise specified.

The room temperature referred to in this specification can mean a temperature in the range of 10 to 40°C.

The notation "Cₙ₋ₘ" (wherein n and m are each a number) used in this specification means that the number of carbon atoms is n or more and m or less, as is usually understood by persons skilled in the art.

In this specification, examples of the "halogen" include a fluorine atom, a chlorine atom, a bromine atom, and an iodine atom.

In this specification, the term "organic group" refers to a group containing at least one carbon atom.

Examples of the "organic group" include alkyl groups optionally having one or more substituents, alkenyl groups optionally having one or more substituents, alkynyl groups optionally having one or more substituents, aryl groups optionally having one or more substituents, aralkyl groups optionally having one or more substituents, non-aromatic heterocyclic groups optionally having one or more substituents, heteroaryl groups optionally having one or more substituents, a cyano group, an aldehyde group, a carboxyl group, R^{r}O-, R^{r}CO-, R^{r}COO-, R^{r}SO₂-, R^{r}OCO-, R^{r}OSO₂- (wherein R^{r} independently represents an alkyl group optionally having one or more substituents, an alkenyl group optionally one or more substituents, an alkynyl group optionally having one or more substituents, an aryl group optionally having one or more substituents, an aralkyl group optionally having one or more substituents, a non-aromatic heterocyclic group optionally having one or more substituents, or a heteroaryl group optionally having one or more substituents).

In this specification, examples of the "hydrocarbon" include alkyl groups, alkenyl groups, alkynyl groups, aryl groups, aralkyl groups, and groups of combinations of these groups.

In this specification, the "alkyl" can be a linear, branched, or cyclic alkyl group.

In this specification, examples of the "alkyl" include C₁₋₂₀ alkyl, C₁₋₁₂ alkyl, C₁₋₆ alkyl, C₁₋₄ alkyl, or C₁₋₃ alkyl.

In this specification, examples of the "alkyl" include linear or branched alkyl groups, such as methyl, ethyl, propyl (n-propyl, isopropyl), butyl (n-butyl, isobutyl, sec-butyl, tert-butyl), pentyl, and hexyl.

In this specification, examples of the "alkyl" include cyclic alkyl or cycloalkyl groups (e.g., C₃₋₈ cycloalkyl groups), such as cyclopropyl, cyclobutyl, cyclopentyl, and cyclohexyl.

In this specification, the "fluoroalkyl" is an alkyl group in which at least one hydrogen atom is replaced with a fluorine atom.

In this specification, the number of fluorine atoms in the "fluoroalkyl" can be 1 or more (e.g., 1 to 3, 1 to 6, 1 to 12, or 1 to the maximum substitutable number).

In this specification, the "fluoroalkyl" can be, for example, C₁₋₂₀ fluoroalkyl, C₁₋₁₂ fluoroalkyl, C₁₋₆ fluoroalkyl, C₁₋₄ fluoroalkyl, or C₁₋₃ fluoroalkyl.

In this specification, the "fluoroalkoxy" can be a linear or branched fluoroalkoxy group.

In this specification, the "fluoroalkoxy" can be a perfluoroalkoxy group or a non-perfluoroalkyl group.

In this specification, examples of the "fluoroalkoxy" include fluoromethyl, difluoromethyl, trifluoromethyl, 2,2,2-trifluoroethyl, pentafluoroethyl, tetrafluoropropyl (e.g., HCF₂CF₂CH₂), hexafluoropropyl (e.g., (CF₃)₂CH-), nonafluorobutyl, octafluoropentyl (e.g., HCF₂CF₂CF₂CF₂CH₂-), and tridecafluorohexyl.

In this specification, the "alkenyl" can be, for example, C₂₋₁₀ alkenyl.

In this specification, examples of the "alkenyl" include linear or branched alkenyl groups such as vinyl, 1-propen-1-yl, 2-propen-1-yl, isopropenyl, 2-butene-1-yl, 4-penten-1-yl, and 5-hexen-1-yl.

In this specification, examples of the "alkenyl" include cyclic alkenyl or cycloalkenyl groups (e.g., C₃₋₈ cycloalkenyl groups) such as cyclopropenyl, cyclobutenyl, cyclopentenyl, cyclohexenyl, and cycloheptenyl.

In this specification, the "alkynyl" can be, for example, a C₂₋₁₀ alkynyl group.

In this specification, examples of the "alkynyl" includes linear or branched alkynyl groups such as ethynyl, 1-propyn-1-yl, 2-propyn-1-yl, 4-pentyn-1-yl, and 5-hexyn-1-yl.

In this specification, the "aryl" can be, for example, monocyclic, bicyclic, tricyclic, or tetracyclic.

In this specification, the "aryl" can be C₆₋₁₈ aryl, C₆₋₁₆ aryl, C₆₋₁₄ aryl, or C₆₋₁₂ aryl.

In this specification, examples of the "aryl" include phenyl, 1-naphthyl, 2-naphthyl, 2-biphenyl, 3-biphenyl, 4-biphenyl, and 2-anthryl.

In this specification, the "aralkyl" can be, for example, C₇₋₁₉ aralkyl, C₇₋₁₇ aralkyl, C₇₋₁₅ aralkyl, or C₇₋₁₃ aralkyl.

In this specification, examples of the "aralkyl" include benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylmethyl, 3-biphenylmethyl, and 4-biphenylmethyl.

In this specification, the "non-aromatic heterocyclic group" can be, for example, monocyclic, bicyclic, tricyclic, or tetracyclic.

In this specification, the "non-aromatic heterocyclic group" can be, for example, a non-aromatic heterocyclic group containing, in addition to carbon atoms, 1 to 4 heteroatoms selected from oxygen, sulfur, and nitrogen as ring-constituting atoms.

In this specification, the "non-aromatic heterocyclic group" may be saturated or unsaturated.

In this specification, examples of the "non-aromatic heterocyclic group" include tetrahydrofuryl, oxazolidinyl, imidazolinyl, aziridinyl, azetidinyl, pyrrolidinyl, piperidinyl, azepanyl, azocanyl, piperazinyl, diazepinyl, diazocanyl, tetrahydropyranyl, morpholinyl, thiomorpholinyl, 2-oxazolidinyl, dihydrofuryl, dihydropyranyl, dihydroquinolyl, and the like.

In this specification, examples of the "heteroaryl" include monocyclic aromatic heterocyclic groups (e.g., 5- or 6-membered monocyclic aromatic heterocyclic groups) and aromatic fused heterocyclic groups (e.g., 5- to 18-membered aromatic fused heterocyclic groups).

In this specification, examples of the "5- or 6-membered monocyclic aromatic heterocyclic groups" include pyrrolyl, furyl, thienyl, pyrazolyl, imidazolyl, isoxazolyl, oxazolyl, isothiazolyl, thiazolyl, triazolyl, oxadiazolyl, thiadiazolyl, tetrazolyl, pyridyl, pyridazinyl, pyrimidinyl, pyrazinyl, and the like.

In this specification, examples of the "5-18 membered aromatic fused heterocyclic group" include isoindolyl, indolyl, benzofuranyl, benzothienyl, indazolyl, benzoimidazolyl, 1,2-benzoisoxazolyl, benzoxazolyl, 1,2-benzoisothiazolyl, benzothiazolyl, isoquinolyl, quinolyl, cinnolinyl, phthalazinyl, quinazolinyl, quinoxalinyl, pyrazolo[1,5-a]pyridyl, imidazo[1,2-a]pyridyl and the like.

In this specification, examples of the "R^{r}O-" include alkoxy groups, cycloalkoxy groups (e.g., C₃₋₈ cycloalkoxy groups such as cyclopentoxy and cyclohexoxy), aryloxy groups (e.g. C₆₋₁₈ aryloxy groups such as phenoxy and naphthoxy), and aralkyloxy groups (e.g., C₇₋₁₉ aralkyloxy groups such as benzyloxy and phenethyloxy).

In this specification, the "alkoxy" can be a group to which an oxygen atom is bound to alkyl(alkyl-O-).

In this specification, the "alkoxy" can be a linear or branched alkoxy group.

In this specification, examples of the "alkoxy" include linear or branched C₁₋₂₀ alkoxy groups such as methoxy, ethoxy, propoxy (n-propoxy, isopropoxy), butoxy (n-butoxy, isobutoxy, sec-butoxy, and tert-butoxy), pentyloxy, and hexyloxy.

In this specification, the "alkylthio" can be a group in which a sulfur atom is bound to alkyl (alkyl-S-).

In this specification, the "alkylthio" can be a linear or branched alkylthio group.

In this specification, examples of the "alkylthio" include linear or branched C₁₋₂₀ alkylthio groups such as methylthio, ethylthio, propylthio (n-propylthio and isopropylthio), butylthio (e.g., n-butylthio, isobutylthio, sec-butylthio, and tert-butylthio), pentylthio, and hexylthio.

In this specification, examples of the "R^{r}CO-" include alkylcarbonyl (e.g., (C₁₋₁₀ alkyl) carbonyl groups such as acetyl, propionyl, and butyryl), arylcarbonyl (e.g., (C₆₋₁₈ aryl) carbonyl groups such as benzoyl and naphthoyl), and aralkyl-carbonyl (e.g., (C₇₋₁₉ aralkyl) carbonyl groups such as benzylcarbonyl and phenethylcarbonyl) .

In this specification, examples of the "R^{r}COO-" include alkylcarbonyloxy (e.g., (C₁₋₁₀ alkyl) carbonyloxy groups such as acetyloxy, propionyloxy, butyryloxy), arylcarbonyloxy (e.g., (C₆₋₁₈ aryl)carbonyloxy groups such as benzoyloxy and naphthoyloxy), and aralkylcarbonyloxy (e.g., (C₇₋₁₉ aralkyl)carbonyloxy groups such as benzylcarbonyloxy and phenethylcarbonyloxy).

In this specification, examples of the "R^{r}SO₂-" include alkylsulfonyl (e.g., C₁₋₁₀ alkylsulfonyl groups such as methylsulfonyl, ethylsulfonyl, and propylsulfonyl), arylsulfonyl (e.g., C₆₋₁₈ arylsulfonyl groups such as phenylsulfonyl and naphthylsulfonyl), and aralkylsulfonyl (e.g., C₇₋₁₉ aralkylsulfonyl groups such as benzylsulfonyl and phenethylsulfonyl).

In the specification, examples of the "R^{r}OCO-" include alkoxycarbonyl (e.g., (C₁₋₁₀ alkoxy) carbonyl groups such as methoxycarbonyl, ethoxycarbonyl, or propoxycarbonyl), aryloxycarbonyl (e.g., (C₆₋₁₈ aryloxy) carbonyl groups such as phenoxycarbonyl and naphthoxycarbonyl), and aralkyloxycarbonyl (e.g., (C₇₋₁₉ aralkyloxy) carbonyl groups such as benzyloxycarbonyl and phenethyloxycarbonyl).

In this specification, examples of the "R^{r}OSO₂-" include alkoxysulfonyl (e.g., C₁₋₁₀ alkoxysulfonyl groups such as methoxysulfonyl, ethoxysulfonyl, and propoxysulfonyl), aryloxysulfonyl (e.g., C₆₋₁₈ aryloxysulfonyl groups such as phenoxysulfonyl and naphthoxysulfonyl), and aralkyloxysulfonyl groups (e.g., C₇₋₁₉ aralkyloxysulfonyl groups such as benzyloxysulfonyl and phenethyloxysulfonyl groups).

In this specification, examples of substituents in the "hydrocarbon group optionally having one or more substituents," "alkyl group optionally having one or more substituents," "alkenyl group optionally having one or more substituents," "alkynyl group optionally having one or more substituents," "aryl group optionally having one or more substituents," "aralkyl group optionally having one or more substituents," "non-aromatic heterocyclic group optionally having one or more substituents," "heteroaryl group optionally having one or more substituents" include a halo group, a nitro group, a cyano group, an oxo group, a thioxo group, a carboxyl group, a sulfo group, a sulfamoyl group, a sulfinamoyl group, a sulfenamoyl group, R^{r}O-, R^{r}CO-, R^{r}COO-, R^{r}SO₂-, R^{r}OCO-, and R^{r}OSO₂- (in these formulas, R^{r} is as defined above).

Of these substituents, examples of the "halo group" can include fluoro, chloro, bromo, and iodo.

The number of substituents can be within the range of 1 to the maximum substitutable number (e.g., 1, 2, 3, 4, 5, or 6).

### Purification Method

In one embodiment, the method for purifying the compound represented by formula (1): 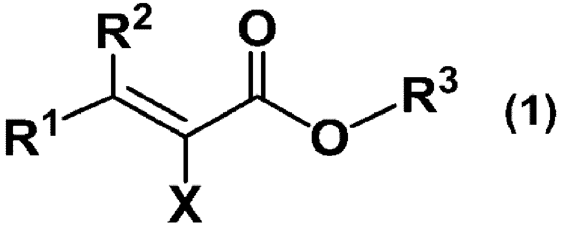 (wherein R¹ and R² are be the same or different and represent an alkyl group, a fluoroalkyl group, an aryl group optionally having one or more substituents, a halogen atom, or a hydrogen atom, R³ represents an alkyl group, a fluoroalkyl group, or an aryl group optionally having one or more substituents, and
X represents a fluoroalkyl group or a halogen atom)
includes the step (A) of mixing
a composition comprising the compound represented by formula (1) and a compound represented by formula (2):

   R⁴-OH (2)

   (wherein R⁴ is an alkyl group, a fluoroalkyl group, or an aryl group optionally having one or more substituents)
with (i) a salt, (ii) a specific organic solvent (with the proviso that the compound represented by formula (1) and the compound represented by formula (2) are excluded from the organic solvent), or (iii) the salt and the organic solvent to obtain a mixture; and
the step (B) of separating the mixture into two or more phases that are different from each other in terms of the content of the compound represented by formula (1).

### Step A

The composition is not limited as long as the composition contains the compound represented by formula (1) and the compound represented by formula (2). The composition can be, for example, a crude product of the compound of formula (1) containing the compound of formula (2) as an impurity.

In formula (1), R¹ is preferably a hydrogen atom, alkyl, or fluoroalkyl, more preferably a hydrogen atom, C₁₋₂₀ alkyl (preferably C₁₋₁₂ alkyl, more preferably C₁₋₆ alkyl, even more preferably C₁₋₄ alkyl, still even more preferably C₁₋₃ alkyl, particularly preferably C₁ or C₂ alkyl), or C₁₋₂₀ fluoroalkyl (preferably C₁₋₁₂ fluoroalkyl, more preferably C₁₋₆ fluoroalkyl, even more preferably C₁₋₄ fluoroalkyl, still even more preferably C₁₋₃ fluoroalkyl, and particularly preferably C₁ or C₂ fluoroalkyl), and even more preferably a hydrogen atom.

In formula (1), R² is preferably a hydrogen atom, alkyl, or fluoroalkyl, more preferably a hydrogen atom, C₁₋₂₀ alkyl (preferably C₁₋₁₂ alkyl, more preferably C₁₋₆ alkyl, even more preferably C₁₋₄ alkyl, still even more preferably C₁₋₃ alkyl, and particularly preferably C₁ or C₂ alkyl, or C₁₋₂₀ fluoroalkyl (preferably C₁₋₁₂ fluoroalkyl, more preferably C₁₋₆ fluoroalkyl, even more preferably C₁₋₄ fluoroalkyl, still even more preferably C₁₋₃ fluoroalkyl, particularly preferably C₁ or C₂ fluoroalkyl) and even more preferably a hydrogen atom.

In formula (1), R³ is preferably an alkyl group, and more preferably a linear alkyl group. R³ is preferably C₁₋₂₀ alkyl, more preferably C₁₋₁₂ alkyl, even more preferably C₁₋₆ alkyl, still even more preferably C₁₋₄ alkyl, and particularly preferably C₁₋₃ alkyl, particularly more preferably methyl or ethyl, and particularly still more preferably methyl.

In formula (1), X is preferably C₁₋₂₀ fluoroalkyl (preferably C₁₋₁₂ fluoroalkyl, more preferably C₁₋₆ fluoroalkyl, even more preferably C₁₋₄ fluoroalkyl, still even more preferably C₁₋₃ fluoroalkyl, and particularly preferably C₁ or C₂ fluoroalkyl), a fluorine atom, or a chlorine atom.

X is more preferably trifluoromethyl, a fluorine atom, or a chlorine atom.

X is even more preferably a fluorine atom or a chlorine atom.

X is particularly preferably a fluorine atom.

In formula (1), R³ is preferably C₁₋₂₀ alkyl (preferably C₁₋₁₂ alkyl, more preferably C₁₋₆ alkyl, even more preferably C₁₋₄ alkyl, still even more preferably C₁₋₃ alkyl, and particularly preferably methyl or ethyl), and X is trifluoromethyl, a fluorine atom, or a chlorine atom.

In formula (1), R³ is more preferably methyl or ethyl (preferably methyl) and X is trifluoromethyl, a fluorine atom, or a chlorine atom.

In formula (1), R¹ is preferably a hydrogen atom, R² is a hydrogen atom, R³ is methyl or ethyl (preferably methyl), and X is a fluorine atom or a chlorine atom.

The compound represented by formula (1) can be produced by a known method or a method similar to the known method, or can be commercially available.

The compound represented by formula (1) can be produced, for example, by the production method described in JPH1-33098 or WO2014/034906, or by methods similar to these methods.

In formula (2), R⁴ is preferably an alkyl group, more preferably a linear alkyl group. R⁴ is preferably C₁₋₂₀ alkyl, more preferably C₁₋₁₂ alkyl, even more preferably C₁₋₆ alkyl, still even more preferably C₁₋₄ alkyl, particularly preferably C₁₋₃ alkyl, particularly more preferably methyl or ethyl, and particularly still more preferably methyl.

R⁴ may be identical to or different from R³ and is preferably identical to R³.

The lower limit of the content of the compound represented by formula (1) in the composition can be preferably 5 mass%, more preferably 10 mass%, and even more preferably 15 mass%.

The upper limit of the content of the compound represented by formula (1) in the composition can preferably be 50 mass%, more preferably 45 mass%, and even more preferably 40 mass%.

The content of the compound represented by formula (1) in the composition can be preferably in the range of 5 to 50 mass%, more preferably in the range of 10 to 45 mass%, and even more preferably in the range of 15 to 40 mass%.

The lower limit of the content of the compound represented by formula (2) in the composition can be preferably 50 mass%, more preferably 55 mass%, and even more preferably 60 mass%.

The upper limit of the content of the compound represented by formula (2) in the composition can be preferably 95 mass%, more preferably 90 mass%, and even more preferably 85 mass%.

The content of the compound represented by formula (2) in the composition can be preferably in the range of 50 to 95 mass%, more preferably in the range of 55 to 90 mass%, and even more preferably in the range of 60 to 85 mass%.

The mass ratio of the compound represented by formula (1) to the compound represented by formula (2) in the composition can be preferably be in the range of 5:95 to 50:50, more preferably in the range of 10:90 to 40:60, and more preferably in the range of 15:85 to 30:70.

The composition may contain one or more other substances in addition to the compound of formula (1) and the compound of formula (2). Examples of such other substances include substances used in the production of the compound represented by formula (1) (e.g., catalysts, bases), by-products, and the like.

The salt (i) is preferably a salt that, when mixed with the composition, can be separated into two or more phases that are different from each other in terms of the content of the compound represented by formula (1). The salt (i) can be at least one salt selected from inorganic salts and organic salts, and preferably an inorganic salt.

The cation of the salt (i) can be, for example, a metal cation, ammonium optionally having one or more substituents, pyridinium optionally having one or more substituents, imidazolium optionally having one or more substituents, or phosphonium optionally having one or more substituents.

Examples of metal cations include monovalent metal cations (e.g., alkali metals such as Li and Na), divalent metal cations (e.g., alkaline earth metals such as Ca), trivalent metal cations (e.g., Group 13 metals of the periodic table such as Al), and the like.

Examples of the ammonium optionally having one or more substituents include NR₄⁺ (wherein the Rs may be the same as or different from each other and represent H or an organic group, and any two of the Rs may be linked together to form a ring optionally having one or more substituents). R is preferably H or a hydrocarbon group (e.g., alkyl and aryl). R is also preferably H or an organic group having 1 to 10 carbon atoms (e.g., C₁₋₁₀ alkyl). It is also preferable that the Rs are all organic groups (e.g., hydrocarbon groups such C₁₋₁₀ alkyl).

Examples of substituents in the pyridinium optionally having one or more substituents include halogen atoms, amino, alkyl, monoalkylamino, dialkylamino, alkylcarbonyl, alkylcarbonylalkyl, aminocarbonyl, aminocarbonylalkyl, cyano, cycloalkyl, aryl, aralkyl, and the like. The number of substituents can be, for example, one, two, or three.

Examples of substituents in the imidazolium optionally having one or more substituents include halogen atoms, alkyl groups, cycloalkyl groups, aryl groups, and aralkyl groups. The number of substituents can be, for example, one, two, or three.

Examples of substituents in the phosphonium optionally having one or more substituents include alkyl, alkenyl, alkoxycarbonylalkyl, monoalkylaminoalkyl, dialkylaminoalkyl, cyanoalkyl, cycloalkyl, aryl, aralkyl, heteroaryl, and the like. The number of substituents can be, for example, one, two, three, or four.

The cation of the salt (i) can be preferably a metal cation.

Examples of the anion of salt (c) include carbonate ions, hydrogen carbonate ions, carboxylate ions, sulfate ions, hydroxide ions, halide ions (e.g. bromide ions, chloride ions, and iodide ions), nitrate ions, and the like.

The anion of the salt (i) can preferably be a halide ion.

The salt (i) is preferably at least one member selected from the group consisting of LiCl, LiBr, LiI, NaI, and CaCl₂.

When the composition is mixed with the salt (i) and water (iv), the lower limit of the amount of the salt (i) used can be preferably 150 mg, and preferably 170 mg, per mL of water.

For example, when the salt (i) is LiCl, the lower limit of the amount of the salt (i) used can preferably be 150 mg, more preferably 170 mg, per mL of water.

When the salt (i) is LiBr, the lower limit of the amount of the salt (i) used can be 310 mg, preferably 350 mg, per mL of water.

When the salt (i) is LiI, the lower limit of the amount of the salt (i) used can be preferably 480 mg, more preferably 500 mg, and even more preferably 540 mg, per mL of water.

When the salt (i) is NaI, the lower limit of the amount of the salt (i) used can be preferably 540 mg, more preferably 550 mg, even more preferably 600 mg, and still even more preferably 610 mg, per mL of water.

When the salt (i) is CaCl₂, the lower limit of the amount of the salt (i) used can be preferably 400 mg and more preferably 450 mg, per mL of water.

The maximum amount of the salt (i) used can be preferably 1360 mg, more preferably 1350 mg, even more preferably 1300 mg, still even more preferably 1250 mg, and particularly preferably 1215 mg, per mL of water.

For example, when the salt (i) is LiCl, the upper limit of the amount of salt (i) used can be 350 mg, preferably 340 mg, per mL of water.

When the salt (i) is LiBr, the upper limit of the amount of the salt (i) used can be preferably 790 mg, more preferably 750 mg, and even more preferably 705 mg per mL of water.

When the salt (i) is LiI, the upper limit of the amount of salt (i) used is preferably 1220 mg, more preferably 1200 mg, even more preferably 1150 mg, still even more preferably 1100 mg, and particularly preferably 1085 mg, per mL of water.

When the salt (i) is NaI, the upper limit of the amount of the salt (i) used can be preferably 1360 mg, more preferably 1350 mg, even more preferably 1300 mg, even more preferably 1250 mg, and particularly preferably 1215 mg, per mL of water.

When the salt (i) is CaCl₂, the upper limit of the amount of the salt (i) used can be preferably 1000 mg, more preferably 950 mg, and even more preferably 900 mg, per mL of water.

The amount of the salt (i) used can be preferably in the range of 150 to 1360 mg, more preferably in the range of 170 to 1215 mg, per mL of water.

For example, when the salt (i) is LiCl, the amount of the salt (i) used can be preferably in the range of 150 to 350 mg, more preferably in the range of 170 to 340 mg, per mL of water.

When the salt (i) is LiBr, the amount of the salt (i) used can be preferably in the range of 310 to 790 mg, more preferably in the range of 350 to 705 mg, per mL of water.

When the salt (i) is LiI, the amount of the salt (i) used can be preferably in the range of 480 to 1220 mg, more preferably 540 to 1085 mg, per mL of water.

When the salt (i) is NaI, the amount of the salt (i) used can preferably be in the range of 540 to 1360 mg, more preferably in the range of 610 to 1215 mg, per mL of water.

When the salt (i) is CaCl₂, the amount of the salt (i) used can be preferably in the range of 400 to 1000 mg, more preferably in the range of 450 to 900 mg, per mL of water.

When the saturated aqueous solution concentration of the salt (i) at room temperature (e.g., 25°C) is defined as A, the salt (i) can be preferably used in an amount to achieve a concentration of 0.5 × A or more, more preferably 0.7 × A or more, and even more preferably 0.8 × A or more. Further, the salt can be preferably used in an amount to achieve a concentration of A or less.

The lower limit of the amount of the salt (i) used is preferably 0.1 moles, more preferably 0.5 moles, per mole of the compound represented by formula (1).

The upper limit of the amount of the salt (i) used can be preferably 10 moles, more preferably 9 moles, per mole of the compound represented by formula (1).

The amount of the salt (i) used is preferably in the range of 0.1 to 10 moles, and more preferably in the range of 0.5 to 9 moles, per mole of the compound represented by formula (1).

The lower limit of the amount of the salt (i) used is preferably 10 parts by mass, more preferably 15 parts by mass, more preferably 20 parts by mass, per 100 parts by mass of the composition.

The upper limit of the amount of the salt (i) used is preferably 100 parts by mass, more preferably 95 parts by mass, even more preferably 90 parts by mass, per 100 parts by mass of the composition.

The amount of the salt (i) used can be preferably in the range of 10 to 100 parts by mass, more preferably in the range of 15 to 95 parts by mass, and even more preferably in the range of 20 to 90 parts by mass, per 100 parts by mass of the composition.

The organic solvent (ii) is not limited as long as the solvent is neither the compound represented by formula (1) nor the compound represented by formula (2). The organic solvent (ii) is preferably a solvent that, when mixed with the composition, separates the resulting mixture into two or more phases that are different from each other in terms of the content of the compound represented by formula (1). The organic solvent (ii) can be, for example, an aprotic solvent. Specific examples of solvents include at least one solvent selected from the group consisting of aliphatic hydrocarbons, aromatic hydrocarbons, halogenated hydrocarbons, ethers, esters (with the proviso that the compound represented by formula (1) is excluded from the esters), ketones, carbonates, and nitriles.

Examples of aliphatic hydrocarbons include C₅₋₁₆ alkanes (e.g., pentane, hexane, heptane, octane, nonane, decane, undecane, dodecane, tridecane, tetradecane, pentadecane, and hexadecane), C₅₋₁₀ cycloalkanes (e.g., cyclopentane and cyclohexane).

Examples of aromatic hydrocarbons include benzene optionally having at least one C₁₋₄ alkyl group. Specific examples include benzene, xylene, toluene, and the like.

Examples of halogenated hydrocarbons include C₁₋₆ haloalkanes (e.g., dichloromethane, dichloroethane, dichloropropane, chlorobutane, and chloroform), benzenes having at least one halogen atom (e.g., chlorobenzene and dichlorobenzene).

Examples of ethers include di(C₁₋₄ alkyl) ethers (e.g., diethyl ether, diisopropyl ether, t-butyl methyl ether, and dibutyl ether), di(C₁₋₄ alkyl) ether of C₂₋₄ alkylene glycol (e.g., monoglyme), di (C₁₋₄ alkyl) ether of poly(C₂₋₄ alkylene glycol) (e.g., diglyme and triglyme), 5-membered oxygen-containing heterocycles (e.g., 1,4-dioxane and tetrahydrofuran), and the like.

Examples of esters (with the proviso that the compound represented by formula (1) is excluded from the esters) include C₁₋₆ alkanoic acid C₁₋₄ alkyl esters. Specific examples include ethyl acetate, butyl acetate, and the like.

Examples of ketones include di(C₁₋₄ alkyl) ketones. Specific examples include methyl ethyl ketone, acetone, and the like.

Examples of carbonates include C₂₋₄ alkylene carbonates. Specific examples include ethylene carbonate, propylene carbonate, and the like.

Examples of nitriles include C₁₋₆ cyanoalkanes (e.g., acetonitrile), benzenes having at least one cyano group (e.g., benzonitrile), and the like.

In one embodiment, the organic solvent (ii) can be preferably an aprotic nonpolar solvent.

In one embodiment, the organic solvent (ii) can be preferably at least one member selected from the group consisting of aromatic hydrocarbons and ethers.

In one embodiment, the organic solvent (ii) is preferably at least one member selected from the group consisting of C₅₋₁₆ alkane, C₅₋₁₀ cycloalkane, benzene optionally having at least one C₁₋₄ alkyl group, C₁₋₆ haloalkane, benzene having at least one halogen atom, di (C₁₋₄ alkyl) ether, di (C₁₋₄ alkyl) ether of C₂₋₄ alkylene glycol, di (C₁₋₄ alkyl) ether of poly(C₂₋₄ alkylene glycol), 5-membered oxygen-containing heterocyclic rings, C₁₋₆ alkanoic acid C₁₋₄ alkyl esters, di (C₁₋₄ alkyl) ketones, C₂₋₄ alkylene carbonate, C₁₋₆ cyanoalkane, and benzene having at least one cyano group.

In this embodiment, the organic solvent (ii) is more preferably pentane, hexane, heptane, octane, octane, nonane, decane, undecane, dodecane, tridecane, tetradecane, pentadecane, hexadecane, cyclopentane, cyclohexane, cyclohexane, benzene, xylene, toluene, dichloromethane, dichloroethane, dichloropropane, chlorobutane, chloroform, chlorobenzene, dichlorobenzene, diethyl ether, diisopropyl ether, t-butyl methyl ether, dibutyl ether, monoglyme, diglyme, triglyme, 1,4-dioxane, tetrahydrofuran, ethyl acetate, butyl acetate, methyl ethyl ketone, acetone, ethylene carbonate, propylene carbonate, acetonitrile, and benzonitrile.

When the organic solvent (ii) alone is mixed with the composition without being combined with the salt (i), water (iv), etc., in order to separate the mixture into two or more phases that are different from each other in terms of the content of the compound represented by formula (1), the organic solvent (ii) preferably has a solubility parameter (SP value) that is, for example, smaller than that of the compound of formula (2), and is preferably 8.2 (cal/cm³)^{1/2} or less.

The solubility parameter can be, for example, a value described in a document (e.g., C.M. Hansen, Ind. Eng. Chem. Prod. Res. Dev., 1969, 8(1), pp. 2-11), or can be an estimated value calculated by the method described in a document (e.g., R.F. Fedors, Polym. Eng. Sci., 1974, 14(2), pp. 147-154).

Examples of organic solvents having a solubility parameter of 8.2 (cal/cm³)^{1/2} or less include, but are not limited to, the following.

| Organic solvent | Solubility parameter (cal/cm³) 1/2 |
|---|---|
| Pentane | 7. 0 |
| Hexane | 7. 3 |
| Heptane | 7. 4 |
| Octane | 7. 5 |
| Nonane | 7. 7 |
| Decane | 7. 7 |
| Undecane | 7. 7 |
| Dodecane | 7. 7 |
| Tridecane | 7. 7 |
| Tetradecane | 7. 7 |
| Pentadecane | 7. 8 |
| Hexadecane | 7. 8 |
| Cyclopentane | 8. 1 |
| Cyclohexane | 8. 2 |
| Diethyl ether | 7. 4 |
| Diisopropyl ether | 7. 8 |
| t-Butyl methyl ether | 7. 9 |

The boiling point of the organic solvent (ii) at normal pressure is preferably 100°C or higher, more preferably 110°C or higher, and even more preferably 120°C or higher. When an organic solvent (ii) having a high boiling point at normal pressure is used, the compound represented by formula (1) can be highly separated from the organic solvent (ii).

The lower limit of the amount of organic solvent (ii) used can be preferably 0.1 moles, more preferably 0.5 moles, and even more preferably 1 mole per mole of the compound represented by formula (1).

The upper limit of the amount of organic solvent (ii) used can be preferably 10 moles, more preferably 5 moles, and even more preferably 2 moles per mole of the compound represented by formula (1).

The amount of the organic solvent (ii) used can be preferably in the range of 0.1 to 10 moles, more preferably in the range of 0.5 to 5 moles, and even more preferably in the range of 1 to 2 moles, per mole of the compound represented by formula (1).

The lower limit of the amount of the organic solvent (ii) used is preferably 30 parts by mass, more preferably 35 parts by mass, and even more preferably 40 parts by mass, per 100 parts by mass of the composition.

The upper limit of the amount of the organic solvent (ii) used is preferably 200 parts by mass, more preferably 150 parts by mass, and even more preferably 100 parts by mass, per 100 parts by mass of the composition.

The amount of the organic solvent (ii) used can be preferably in the range of 30 to 200 parts by mass, more preferably in the range of 35 to 150 parts by mass, even more preferably in the range of 40 to 100 parts by mass, per 100 parts by mass of the composition.

When the salt (i) and the organic solvent (ii) are used in combination, a higher degree of removal of the compound represented by formula (2) can be achieved. The types and amounts of the salt (i) and the organic solvent (ii) used can be the same as those described above.

Step A is preferably a step of mixing the composition with the salt (i) and/or the organic solvent (ii), and water (iv) to obtain a mixture.

The lower limit of the amount of water used can be preferably 10 parts by mass, more preferably 15 parts by mass, and even more preferably 20 parts by mass, per 100 parts by mass of the composition.

The upper limit of the amount of water used can be preferably 200 parts by mass, more preferably 150 parts by mass, and even more preferably 100 parts by mass, per 100 parts by mass of the composition.

The amount of water used can be in the range of 10 to 200 parts by mass, preferably 15 to 150 parts by mass, and even more preferably 20 to 100 parts by mass, per 100 parts by mass of the composition.

Water may be added separately from the salt (i) and/or the organic solvent (ii) to the composition and mixed, or water may be added to the composition together with the salt (i) and/or the organic solvent (ii) (for example, in the form of an aqueous solution when the salt (i) is used) and mixed.

Step A can be preferably performed within the range of -15 to 40°C, more preferably within the range of -15 to 35°C, even more preferably within the range of -15 to 30°C, still even more preferably within the range of -15 to 20°C, particularly preferably within the range of -15 to 15°C, particularly more preferably within the range of -15 to 10°C, and most preferably within the range of -15 to 5°C.

### Step B

Step B is not limited as long as the mixture obtained in step A can be separated into two or more phases that are different from each other in terms of the content of the compound represented by formula (1). Step B may be carried out continuously or batchwise, in a single stage or in multiple stages; and a common method, such as liquid separation, countercurrent contact, or a method using a centrifuge such as a decanter, can be used.

In one embodiment, it is preferred that the mixture obtained in step A is separated into an upper phase and a lower phase. Depending on, for example, the type of organic solvent (ii) and whether water (iv) is used, the phase having a high content of the compound represented by formula (1) may be the upper phase or the lower phase.

In one embodiment, it is preferred that the mixture obtained in step A is separated by specific gravity, and that the mixture obtained in step A is separated into a low-specific-gravity phase and a high-specific-gravity phase. In this embodiment, the low-specific-gravity phase may be a phase having a high content of the compound represented by formula (1), or the high-specific-gravity phase may be a phase having a high content of the compound represented by formula (1). For example, when the composition is mixed with an aprotic solvent having a lower specific gravity than water (e.g., xylene) and water, the upper phase (aprotic solvent phase) may be a phase having a high content of the compound represented by formula (1), and the lower phase (aqueous phase) may be a phase having a low content of the compound represented by formula (1). When the composition is mixed with an aprotic solvent having a higher specific gravity than water (e.g., dichloromethane) and water, the upper phase (aqueous phase) may be a phase having a low content of the compound represented by formula (1), and the lower phase (aprotic solvent phase) may be a phase having a high content of the compound represented by formula (1).

Further, when the composition is mixed with the salt (i), the upper phase may be a phase having a high content of the compound represented by formula (1), and the lower phase may be a phase having a low content of the compound represented by formula (1) .

In one embodiment, it is preferred that the mixture obtained in step A is separated into an organic phase and an aqueous phase. In this embodiment, the organic phase is a phase having a high content of the compound represented by formula (1). The organic phase may be the upper phase or the lower phase.

In one embodiment, it is preferred that the mixture obtained in step A is separated by polarity and that the mixture obtained in step A is separated into a low-polarity phase and a high-polarity phase. In this embodiment, the low-polarity phase is a phase having a high content of the compound represented by formula (1). The low-polarity phase may be the upper phase or the lower phase.

The amount of the compound represented by formula (1) (the content ratio of the compound represented by formula (1)) relative to the total amount of the compound represented by formula (1) and the compound represented by formula (2) in the phase having the highest content of the compound represented by formula (1) (e.g., the low-specific-gravity phase or the high-specific-gravity phase, the low-polarity phase, or the organic phase) can be higher than the content ratio of the compound represented by formula (1) in the composition. In the phase, the mass ratio of the compound represented by formula (1) and the compound represented by formula (2) may be preferably within the range of 80:20 to 99.9:0.1, and more preferably within the range of 85:15 to 99:1.

In a phase other than the above (e.g., the high-specific-gravity phase or the low-specific-gravity phase, the high-polarity phase, or the aqueous phase), the mass ratio of the compound represented by formula (1) and the compound represented by formula (2) may be preferably within the range of 0.1:99.1 to 10:90, and more preferably within the range of 1:99 to 8:92. The method of the present disclosure is also excellent in terms of yield with little loss of the compound represented by formula (1) .

Step B may be performed in the same temperature range as that in step A.

### Step C

The method for purifying the compound represented by formula (1) preferably comprises the following step:
(C) removing a phase that has the lowest content of the compound represented by formula (1) of the separated phases (or a phase other than a phase that has the highest content of the compound represented by formula (1)), or recovering the phase that has the highest content of the compound represented by formula (1) (or a phase other than the phase that has the lowest content of the compound represented by formula (1)). The method of the present disclosure can increase the rate of transfer of the compound represented by formula (2) to the phase having the lowest content of the compound represented by formula (1) (e.g., the high-polarity phase, the aqueous phase) and can remove the compound represented by formula (2) to a high degree.

Step C may be performed in the same temperature range as that in step A.

### Optional Additional Steps

The method for purifying the compound represented by formula (1) may further comprise additional steps.

In one embodiment, the method for purifying the compound represented by formula (1) may further comprise the following steps:
(D) mixing the phase removed in step C with a salt (i) and/or an organic solvent (ii), and optionally water (iv), to obtain a mixture; and
(E) separating the mixture obtained in step D into two or more phases that are different from each other in terms of the content of the compound represented by formula (1).

In this embodiment, the method for purifying the compound represented by formula (1) may further comprise the following step:
(F) removing a phase that has the lowest content of the compound represented by formula (1) of the phases separated in step E (or a phase other than a phase that has the highest content of the compound represented by formula (1)), or recovering the phase that has the highest content of the compound represented by formula (1) (or a phase other than the phase that has the lowest content of the compound represented by formula (1)).

Step D, step E, and step F can be performed in the same manner as in step A, step B, and step C, respectively.

Steps D to F are steps of recovering the compound represented by formula (1) in the phase removed in step C. The series of steps D to F may be repeated by using, in step D, the phase removed in step F in place of the phase removed in step C.

The method for purifying the compound represented by formula (1) may comprise the following step in addition to steps D to F:
(G) mixing the phase obtained in step C with the phase obtained in step F.

In one embodiment, the method for purifying the compound represented by formula (1) may comprise the following step:
(H) concentrating the phase obtained in step C (or the phase obtained in step F or step G).

The concentration method in step H is not limited as long as the content of the compound represented by formula (1) can be increased, and examples of the concentration method include distillation under reduced pressure and the like.

The content of the organic solvent (ii) in the concentrate may be preferably 5 mass% or less, more preferably 3 mass% or less, and even more preferably 1 mass% or less.

In one embodiment, the method for purifying the compound represented by formula (1) may further comprise the following step:
(I) recovering the salt (i) and/or the organic solvent (ii) used for purification.
The recovered salt (i) and/or organic solvent (ii) may be reused in step A and/or step D.

### Composition

In one embodiment, the composition is a composition comprising a compound represented by formula (1), a compound represented by formula (2), and a salt (i), wherein the content of the salt (i) is 2 mass% or less (hereinafter referred to as "composition a").

The content of the salt (i) in composition a may be preferably 1 mass% or less, and more preferably 0.5 mass% or less. The content of the salt (i) in composition a may be, for example, at or above the detection limit.

In composition a, the mass ratio of the compound represented by formula (1) and the salt (i) may be preferably within the range of 25:1 to 160:1, more preferably within the range of 30:1 to 120:1, and even more preferably within the range of 40:1 to 80:1.

In composition a, the mass ratio of the compound represented by formula (1) and the compound represented by formula (2) may be preferably within the range of 80:20 to 99.9:0.1, and more preferably 85:15 to 99:1.

Composition a may further comprises an organic solvent (ii). The content of the organic solvent (ii) in composition a may be, for example, 20 mass% or less or may be, for example, 20 mass% or more, 25 mass% or more, or 30 mass% or more.

In composition a, the compound represented by formula (1), the compound represented by formula (2), the salt (i), and the organic solvent (ii) may each be selected from those described in the "Purification Method" section above.

Composition a may be produced, for example, by a method comprising steps A to C and optional steps D to G described in the "Purification Method" section above.

In another embodiment, the composition is a composition comprising a compound represented by formula (1) and an organic solvent (ii), wherein the content of the organic solvent (ii) is 20 mass% or less (hereinafter referred to as "composition b").

The content of the organic solvent (ii) in composition b may be preferably 15 mass% or less, more preferably 10 mass% or less, even more preferably 5 mass% or less, and still even more preferably 1 mass% or less. The content of the organic solvent (ii) in composition b may be, for example, at or above the detection limit.

Composition b may further comprise a compound represented by formula (2). In this case, the mass ratio of the compound represented by formula (1) and the compound represented by formula (2) may be preferably within the range of 80:20 to 99.9:0.1, and more preferably within the range of 85:15 to 99:1.

In composition b, the compound represented by formula (1), the compound represented by formula (2), and the organic solvent (ii) may each be selected from those described in the "Purification Method" section above.

Composition b may be produced by a method comprising steps A to C, optional steps D to G, and step H described in the "Purification Method" section above, or a method of concentrating composition a.

### Examples

One embodiment according to the present disclosure is described below in more detail with reference to Examples. However, the present disclosure is not limited to the Examples.

### Synthesis Example 1

Monofluorinated acrylic acid methyl ester was synthesized according to the methods described in the Example and Reference Example of JPH01-033098B.

### Example 1

(I) To 20 g of a mixed solution of 20 mass% of the monofluorinated acrylic acid methyl ester obtained in Synthesis Example 1 and 80 mass% of methanol, 10.5 g of a 47 mass% calcium chloride aqueous solution and 10.5 g of xylene were added at 0°C. After sufficient stirring, an upper phase and a lower phase were individually separated. The amount of the upper phase was 13 g, and the amount of the lower phase was 28 g.

Analysis of the upper phase by GC, NMR, the Karl Fischer method, and elemental analysis showed that the upper phase had the following composition.

### Upper phase

| | |
|---|---|
| Monofluorinated acrylic acid methyl ester | 24 mass% |
| Methanol | 3 mass% |
| Xylene | 73 mass% |
| Water | 568 ppm |
| Ca | <5 ppm |

The lower phase was also analyzed in the same manner, and the results showed that the lower phase had the following composition.

### Lower phase

| | |
|---|---|
| Monofluorinated acrylic acid methyl ester | 3 mass% |
| Methanol | 56 mass% |
| Xylene | 4 mass% |
| Water | 20 mass% |
| Ca | 17 mass% |

(II) To the lower phase obtained in (I) above, 17 g of xylene was added at 0°C. After sufficient stirring, an upper phase and a lower phase were individually separated. The amount of the upper phase was 18 g, and the amount of the lower phase was 27 g.

Analysis of the upper phase by GC, NMR, the Karl Fischer method, and elemental analysis showed that the upper phase had the following composition.

### Upper phase

| | |
|---|---|
| Monofluorinated acrylic acid methyl ester | 4.6 mass% |
| Methanol | 1.4 mass% |
| Xylene | 94 mass% |
| Water | 304 ppm |
| Ca | <5 ppm |

Analysis of the lower phase by GC showed that the lower phase had the following composition.

### Lower phase

| | |
|---|---|
| Monofluorinated acrylic acid methyl ester | 2 mass% |
| Methanol | 98 mass% |

(III) The upper phase obtained in (I) above was mixed with the upper phase obtained in (II) above to obtain a mixture having the following composition.

### Mixture

| | |
|---|---|
| Monofluorinated acrylic acid methyl ester | 13 mass% |
| Methanol | 2 mass% |
| Xylene | 85 mass% |
| Water | 872 ppm |
| Ca | <5 ppm |

(The mass ratio of monofluorinated acrylic acid methyl ester and methanol is 87:13.)

(IV) The mixed solution obtained in (III) above was distilled under reduced pressure. A distillate was obtained with 99% recovery of monofluorinated acrylic acid methyl ester. Analysis of the distillate by GC and NMR showed that the distillate had the following composition.

### Distillate

| | |
|---|---|
| Monofluorinated acrylic acid methyl ester | 84 mass% |
| Methanol | 15 mass% |
| Xylene | 1 mass% |

### Composition of still residue

| | |
|---|---|
| Monofluorinated acrylic acid methyl ester | 2 mass% |
| Methanol | 1 mass% |
| Xylene | 97 mass% |

### Example 2

To 20 g of a mixed solution of 30 mass% of the monofluorinated acrylic acid methyl ester obtained in Synthesis Example 1 and 70 mass% of methanol, 12 g of a 47 mass% calcium chloride aqueous solution was added at 0°C. After sufficient stirring, an upper phase and a lower phase were individually separated.

Analysis of the upper phase by GC, NMR, the Karl Fischer method, and elemental analysis showed that the upper phase had the following composition.

### Upper phase

| | |
|---|---|
| Monofluorinated acrylic acid methyl ester | 92 mass% |
| Methanol | 8 mass% |
| Ca | <5 ppm |

Analysis of the lower phase by GC showed that the lower phase had the following composition.

### Lower phase

| | |
|---|---|
| Monofluorinated acrylic acid methyl ester | 4 mass% |
| Methanol | 60 mass% |
| Water | 28 mass% |
| Ca | 8 mass% |

### Comparative Example 1

To 20 g of a mixed solution of 20 mass% of the monofluorinated acrylic acid methyl ester obtained in Synthesis Example 1 and 80 mass% of methanol, 10.5 g of water was added, and the mixture was stirred to obtain a one-phase solution.

### Example 3

As shown in Table 1, a 47 mass% calcium chloride aqueous solution and an extraction solvent were added to a mixed solution (crude product) of 7.68 mass% of the monofluorinated acrylic acid methyl ester obtained in Synthesis Example 1, 90.33 mass% of methanol (MeOH), 0.04 mass% of methyl fluoroacetate, 0.02 mass% of dimethyl carbonate (DMC), and 1.93 mass% of triethylamine (TEA). After sufficient stirring, an upper phase and a lower phase were individually separated. The composition of each of the upper phase and the lower phase was analyzed by GC, NMR, the Karl Fischer method, and elemental analysis. Table 2 shows the composition of each phase (excluding the extraction solvent).

**Table 1**

| | Amount of aqueous solution added (V) | Extraction solvent | Amount of solvent added (V) | Extraction temperature |
|---|---|---|---|---|
| 3-1 | 0.8 | Cetane | 0.3 | Room temperature |
| 3-2 | 0.8 | Xylene | 0.5 | Room temperature |
| 3-3 | 0.8 | Xylene (first extraction) | 0.5 | 0°C |
| 3-4 | 0.8 | Xylene (second extraction) | 0.3 | 0°C |
| 3-5 | 0.8 | Xylene (first extraction) | 0.5 | 0°C |
| 3-6 | 0.8 | Xylene (second extraction) | 0.4 | 0°C |
| 3-7 | 0.8 | Xylene (third extraction) | 0.3 | 0°C |
| 3-8 | 0.8 | Dichloromethane | 0.5 | 0°C |
| 3-9 | 0.8 | Dibutyl ether | 0.5 | 0°C |

**Table 2**

| | | MeOH (mass%) | Methyl fluoroacetate (mass%) | DMC (mass%) | Monofluorinated acrylic acid methyl ester (mass%) | TEA (mass%) | Water (mass%) | Ca (mass%) |
|---|---|---|---|---|---|---|---|---|
| 3-1 | Upper phase | 2.68 | 0.05 | 0.08 | 96.54 | 0.5 | 0.15 | N.D. |
| | Lower phase | 51.92 | 0.01 | 0.01 | 1.09 | N.D. | 34.81 | 11.32 |
| 3-2 | Upper phase | 8.94 | 0.11 | 0.11 | 90.64 | N.D. | 0.14 | N.D. |
| | Lower phase | 51.96 | 0.01 | 0.01 | 0.89 | N.D. | 35.46 | 11.57 |
| 3-3 | Upper phase | 4.73 | 0.11 | 0.12 | 94.71 | N.D. | 0.21 | N.D. |
| | Lower phase | 52.07 | 0.02 | 0.01 | 0.85 | N.D. | 35.41 | 11.55 |
| 3-4 | Upper phase | 9.37 | 0.20 | 0.19 | 89.90 | N.D. | 0.19 | N.D. |
| | Lower phase | 51.74 | 0.01 | 0.01 | 0.26 | N.D. | 35.70 | 11.51 |
| 3-5 | Upper phase | 3.88 | N.D. | N.D. | 95.55 | 0.29 | 0.19 | N.D. |
| | Lower phase | 59.05 | N.D. | N.D. | 0.59 | 0.31 | 35.39 | 11.58 |
| 3-6 | Upper phase | 9.37 | N.D. | N.D. | 90.36 | N.D. | 0.20 | N.D. |
| | Lower phase | 52.33 | N.D. | N.D. | 0.14 | 0.30 | 35.50 | 11.61 |
| 3-7 | Upper phase | 21.50 | N.D. | N.D. | 78.16 | N.D. | 0.20 | N.D. |
| | Lower phase | 52.15 | N.D. | N.D. | 0.04 | 0.08 | 35.76 | 11.63 |
| 3-8 | Upper phase | 52.07 | N.D. | N.D. | 1.09 | N.D. | 35.08 | 11.68 |
| | Lower phase | 14.77 | N.D. | N.D. | 84.93 | N.D. | 0.12 | N.D. |
| 3-9 | Upper phase | 12.19 | N.D. | N.D. | 87.57 | N.D. | 0.15 | N.D. |
| | Lower phase | 51.85 | N.D. | N.D. | 1.74 | N.D. | 34.13 | 11.57 |

### Example 4

As shown in Table 3, a mixed solution (crude product) of 29.5 mass% of the monofluorinated acrylic acid methyl ester obtained in Synthesis Example 1, 69.1 mass% of methanol, and 1.4 mass% of triethylamine (TEA) was collected in a screw tube, and an inorganic salt was added and dissolved by shaking. An extraction solvent was added to the resulting solution, and the mixture was shaken for extraction. The composition of each of an upper phase and a lower phase was analyzed by GC, NMR, the Karl Fischer method, and elemental analysis. Table 4 shows the composition of each phase (excluding the extraction solvent).

**Table 3**

| | Amount of crude product (g) | Inorganic salt | Amount of salt added (mass%) | Extraction solvent | Amount of solvent added (V) | Extraction temperature |
|---|---|---|---|---|---|---|
| 4-1 | 10 | CaCl₂ | 20 | Xylene | 0.5 | Room temperature |
| 4-2 | 10 | LiBr | 20 | Xylene | 0.5 | Room temperature |
| 4-3 | 10 | LiCl | 10 | Xylene | 0.7 | Room |
| | | | | | | temperature |
| 4-4 | 20 | CaCl₂ | 20 | Xylene | 0.3 | -15°C |
| 4-5 | 20 | CaCl₂ | 20 | Xylene | 0.8 | -15°C |
| 4-6 | 20 | CaCl₂ | 20 | Xylene | 1.2 | -15°C |
| 4-7 | 20 | CaCl₂ | 20 | Xylene | 1.8 | -15°C |
| 4-8 | 7 | CaCl₂ | 20 | Dibutyl ether | 1.0 | Room temperature |
| 4-9 | 7 | CaCl₂ | 20 | Dibutyl ether | 1.0 | -15°C |

**Table 4**

| | | MeOH (mass%) | Monofluorinated acrylic acid methyl ester (mass% ) | TEA (mass%) | Ca or Li (mass%) |
|---|---|---|---|---|---|
| 4-1 | Upper phase | 21.4 | 74.0 | 2.0 | 2.2 |
| | Lower phase | 73.7 | 18.5 | - | 7.7 |
| 4-2 | Upper phase | 25.8 | 68.7 | 4.6 | 0.6 |
| | Lower phase | 74.3 | 21.5 | 2.4 | 1.7 |
| 4-3 | Upper phase | 17.4 | 17.4 | 6.9 | 0.4 |
| | Lower phase | 79.6 | 79.6 | 1.9 | 1.9 |
| 4-4 | Upper phase | 14.9 | 83.0 | - | 1.8 |
| | Lower phase | 66.8 | 23.9 | 1.0 | 8.2 |
| 4-5 | Upper phase | 20.5 | 77.0 | 0.1 | 2.2 |
| | Lower phase | 79.1 | 11.7 | 0.9 | 8.3 |
| 4-6 | Upper phase | 20.8 | 76.8 | - | 2.2 |
| | Lower phase | 82.5 | 8.6 | 0.3 | 8.7 |
| 4-7 | Upper phase | 21.1 | 75.8 | 0.6 | 2.2 |
| | Lower phase | 84.3 | 6.4 | 0.4 | 8.8 |
| 4-8 | Upper phase | 31.5 | 62.3 | 2.6 | 3.3 |
| | Lower phase | 74.1 | 17.5 | 0.6 | 7.7 |
| 4-9 | Upper phase | 29.3 | 65.6 | 1.9 | 3.1 |
| | Lower phase | 78.9 | 12.1 | 0.7 | 8.3 |

## Claims

1. A method for purifying a compound represented by formula (1): 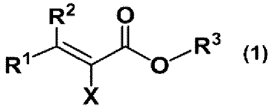 wherein
R¹ and R² are the same or different and are an alkyl group, a fluoroalkyl group, an aryl group optionally having one or more substituents, a halogen atom, or a hydrogen atom,
R³ is an alkyl group, a fluoroalkyl group, or an aryl group optionally having one or more substituents, and
X is a fluoroalkyl group or a halogen atom, the method comprising
step (A) of mixing
a composition comprising
the compound represented by formula (1) and
a compound represented by formula (2):
R⁴-OH (2)
wherein R⁴ is an alkyl group, a fluoroalkyl group, or an aryl group optionally having one or more substituents
with
(i) a salt,
(ii) an organic solvent, with the proviso that the compound represented by formula (1) and the compound represented by formula (2) are excluded from the organic solvent, or
(iii) the salt and the organic solvent to obtain a mixture; and
step (B) of separating the mixture into two or more phases that are different from each other in terms of the content of the compound represented by formula (1).

2. The method according to claim 1, wherein the salt is at least one member selected from the group consisting of inorganic salts and organic salts.

3. The method according to claim 1 or 2, wherein the salt is an inorganic salt.

4. The method according to any one of claims 1 to 3, wherein the salt comprises at least one cation selected from the group consisting of metal cations, ammonium optionally having one or more substituents, pyridinium optionally having one or more substituents, imidazolium optionally having one or more substituents, and phosphonium optionally having one or more substituents.

5. The method according to any one of claims 1 to 4, wherein the salt comprises at least one cation selected from the group consisting of monovalent metal cations and divalent metal cations.

6. The method according to any one of claims 1 to 4, wherein the salt comprises a cation of NR₄⁺, wherein each R may be the same or different and is H or a C₁₋₁₀ organic group.

7. The method according to any one of claims 1 to 6, wherein the salt comprises at least one anion selected from the group consisting of sulfate ions, hydroxide ions, halide ions, and nitrate ions.

8. The method according to any one of claims 1 to 5, wherein the salt is at least one member selected from the group consisting of LiCl, LiBr, LiI, NaI, and CaCl₂.

9. The method according to any one of claims 1 to 8, wherein the salt is used in an amount of 0.1 to 10 moles per mole of the compound represented by formula (1).

10. The method according to any one of claims 1 to 9, wherein the organic solvent is an aprotic solvent, with the proviso that the compound represented by formula (1) and the compound formula (2) are excluded from the aprotic solvent.

11. The method according to any one of claims 1 to 10, wherein the organic solvent is an aprotic nonpolar solvent, with the proviso that the compound represented by formula (1) and the compound represented by formula (2) are excluded from the aprotic nonpolar solvent.

12. The method according to any one of claims 1 to 10, wherein the organic solvent is at least one member selected from the group consisting of aliphatic hydrocarbons, aromatic hydrocarbons, halogenated hydrocarbons, ethers, esters, ketones, carbonates, and nitriles, with the proviso that the compound represented by formula (1) is excluded from the esters.

13. The method according to any one of claims 1 to 10, wherein the organic solvent is at least one member selected from the group consisting of aromatic hydrocarbons and ethers.

14. The method according to any one of claims 1 to 10, wherein the organic solvent is at least one member selected from the group consisting of C₅₋₁₆ alkane, C₅₋₁₀ cycloalkane, benzene optionally having at least one C₁₋₄ alkyl, C₁₋₆ haloalkane, benzene having at least one halogen atom, di (C₁₋₄ alkyl) ether, di (C₁₋₄ alkyl) ether of C₂₋₄ alkylene glycol, di (C₁₋₄ alkyl) ether of poly(C₂₋₄ alkylene glycol), 5-membered oxygen-containing heterocyclic rings, C₁₋₆ alkanoic acid C₁₋₄ alkyl esters, di (C₁₋₄ alkyl) ketone, C₂₋₄ alkylene carbonate, C₁₋₆ cyanoalkane, and benzene having at least one cyano group.

15. The method according to any one of claims 1 to 10, wherein the organic solvent is at least one member selected from the group consisting of pentane, hexane, heptane, octane, nonane, decane, undecane, dodecane, tridecane, tetradecane, pentadecane, hexadecane, cyclopentane, cyclohexane, benzene, xylene, toluene, dichloromethane, dichloroethane, dichloropropane, chlorobutane, chloroform, chlorobenzene, dichlorobenzene, diethyl ether, diisopropyl ether, t-butyl methyl ether, dibutyl ether, monoglyme, diglyme, triglyme, 1,4-dioxane, tetrahydrofuran, ethyl acetate, butyl acetate, methyl ethyl ketone, acetone, ethylene carbonate, propylene carbonate, acetonitrile, and benzonitrile.

16. The method according to any one of claims 1 to 15, wherein the organic solvent is used in an amount of 0.1 to 10 moles per mole of the compound represented by formula (1).

17. The method according to any one of claims 1 to 16, wherein the step (A) is a step of mixing the composition with
(i) a salt,
(ii) an organic solvent, with the proviso that the compound represented by formula (1) and the compound represented by formula (2) are excluded from the organic solvent, or
(iii) the salt and the organic solvent, and
(iv) water
to obtain a mixture.

18. The method according to claim 17, wherein the salt is used in an amount of 150 mg or more per mL of water.

19. The method according to claim 17 or 18, wherein the salt is LiCl, LiBr, LiI, NaI, or CaCl₂;
when the salt is LiCl, the salt is used in an amount of 150 mg or more per mL of water;
when the salt is LiBr, the salt is used in an amount of 310 mg or more per mL of water;
when the salt is LiI, the salt is used in an amount of 480 mg or more per mL of water;
when the salt is NaI, the salt is used in an amount of 540 mg or more per mL of water; and
when the salt is CaCl₂, the salt is used in an amount of 450 mg or more per mL of water.

20. The method according to any one of claims 1 to 19, further comprising step (C) of removing a phase that has the lowest content of the compound represented by formula (1) of the separated phases.

21. The method according to any one of claims 1 to 20, which is performed at a temperature of -15 to 40°C.

22. The method according to any one of claims 1 to 21, wherein R¹ is a hydrogen atom, an alkyl group, or a fluoroalkyl group.

23. The method according to any one of claims 1 to 22, wherein R² is a hydrogen atom, an alkyl group, or a fluoroalkyl group.

24. The method according to any one of claims 1 to 23, wherein R³ is an alkyl group.

25. The method according to any one of claims 1 to 24, wherein R³ is a C₁₋₄ alkyl group.

26. The method according to any one of claims 1 to 25, wherein R⁴ is an alkyl group.

27. The method according to any one of claims 1 to 26, wherein R⁴ is a C₁₋₄ alkyl group.

28. The method according to any one of claims 1 to 27, wherein X is a fluorine atom or a chlorine atom.

29. A composition comprising
a compound represented by formula (1): 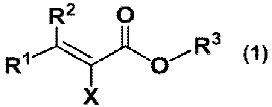 wherein
R¹ and R² are the same or different and are an alkyl group, a fluoroalkyl group, an aryl group optionally having one or more substituents, a halogen atom, or a hydrogen atom,
R³ is an alkyl group, a fluoroalkyl group, or an aryl group optionally having one or more substituents, and
X is a fluoroalkyl group or a halogen atom;
a compound represented by formula (2):
R⁴-OH (2)
wherein R⁴ is an alkyl group, a fluoroalkyl group, or an aryl group optionally having one or more substituents; and
a salt, the content of the salt being 2 mass% or less.

30. A composition comprising
a compound represented by formula (1): 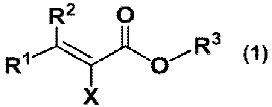 wherein
R¹ and R² are the same or different and are an alkyl group, a fluoroalkyl group, an aryl group optionally having one or more substituents, a halogen atom, or a hydrogen atom,
R³ is an alkyl group, a fluoroalkyl group, or an aryl group optionally having one or more substituents, and
X is a fluoroalkyl group or a halogen atom; and
at least one organic solvent selected from the group consisting of aliphatic hydrocarbons, aromatic hydrocarbons, halogenated hydrocarbons, ethers, esters, ketones, carbonates, and nitriles, with the proviso that the compound represented by formula (1) is excluded from the esters,
the content of the organic solvent being 20 mass% or less.
